Europäisches Patentamt

European Patent Office

Office européen des brevets

(11) Publication number: **0 055 823**
**B1**

(12) **EUROPEAN PATENT SPECIFICATION**

(45) Date of publication of patent specification: **18.09.85**

(21) Application number: **81109643.7**

(22) Date of filing: **12.11.81**

(51) Int. Cl.⁴: **B 65 D 85/00, A 61 B 17/06**

(54) **Package for surgical sutures.**

(30) Priority: **12.12.80 US 215598**

(43) Date of publication of application:
**14.07.82 Bulletin 82/28**

(45) Publication of the grant of the patent:
**18.09.85 Bulletin 85/38**

(84) Designated Contracting States:
**BE CH DE FR GB IT LI NL SE**

(56) References cited:
**DE-A-2 536 924**
**DE-A-2 914 480**
**US-A-4 089 410**
**US-A-4 120 395**

(73) Proprietor: **AMERICAN CYANAMID COMPANY**
**1937 West Main Street P.O. Box 60**
**Stamford Connecticut 06904 (US)**

(72) Inventor: **Kubas, Robert J.**
**15 Aspen Ledges Road**
**Ridgefield Connecticut (US)**

(74) Representative: **Wächtershäuser, Günter, Dr.**
**Tal 29**
**D-8000 München 2 (DE)**

Courier Press, Leamington Spa, England.

## Description

This invention relates to a package for a surgical needle suture, and, more particularly, to a surgical suture package according to the preamble of the main claim.

This prior art is known from U.S.—A—41 20 395. The known package does not permit a direct dispensing of the suture, i.e. the package must be unfolded to allow the suture to be withdrawn.

Other suture packages of the non-direct dispensing type are known from DE—A—29 14 480 and DE—A—25 36 924. These prior art suture packages do not allow the dispensing of a suture without the need for manipulating the package or suture holder.

To reduce the time of operative procedures and to permit surgeons to utilize their skills more effectively, it has become common practice to package surgical tools and appliances so that they are readily accessible to operating room personnel. In addition to using packaging techniques that permit the operating room nurse and the surgeon to manipulate the various surgical devices, the devices are packaged in a sterile environment, so that they are immediately available for use.

In conformance, it has become common practice to package and store surgical needles and sutures in sterile packages. These packages are designed to permit sterilizing the contents, and storing surgical needles and sutures in sterile packages.

The suture packages are designed to permit sterilizing the contents and maintaining the contents in a sterile condition until they are removed for use.

This invention is concerned with a holding device for mounting a suture that protects the point of the needle, acts as a holding device to permit manipulation of the suture, and forms a support on which the suture is organized to avoid tangles, snags or permanent deformation yet allows rapid removal of the suture without the need for manipulating the holder.

The problems of prior art surgical suture packages are overcome by a direct dispensing surgical suture package according to the present invention. The package is characterized by a foam receptacle fixed adjacent to the perimeter of at least one quadrant of the center panel and at least a first flap having a smaller perimeter than the perimeter of said panel, allowing said needle engaged in said receptacle and optionally said loop contained by said receptacle to be accessible without moving said flap relative to said panel whereby said suture is directly dispensed from said package by disengaging and pulling one of said needles, optionally after severing said loop.

In one embodiment, the package has a first receptacle engaging the needles and a second receptacle containing the loop. In another embodiment, the package has a slit on the second receptacle for containing the loop. In yet another embodiment, the package has a slit on the panel to contain a distal corner of the flap.

In still another embodiment, the package has a second flap adjacent the second receptacle. The second flap contains an opening and the first flap has a recess to allow the loop to be accessible and to be severed without unfolding the flaps. In yet another embodiment, the package has a third flap opposite the first flap. The third flap contains a recess to allow the loop to be accessible and to be severed without unfolding the flaps. The third flap is folded onto the second flap and partially onto the first flap. In still yet another embodiment, the package has a slit on the first flap to contain a portion of a distal edge of the third flap.

A direct dispensing surgical suture package comprising a center panel; at least one foam receptacle having a slit, the receptacle affixed to the panel; a double-armed surgical suture with the needles engaged in and having a loop in about the midpoint of the suture contained by the receptacle slit; and at least a first flap adjacent and placed onto the panel allowing the receptacle to be accessible has also been invented. A direct dispensing surgical suture package comprising a center panel; at least one foam receptacle affixed to the panel; a single-armed surgical suture with the needle engaged in the receptacle; and at least a first flap adjacent and placed onto the panel allowing the receptacle to be accessible whereby the suture is dispensed from the package by disengaging and then pulling the needle, has further been invented. In one embodiment, either package has a slit on the panel to contain a distal corner of the flap. In another embodiment, either package has a second flap adjacent the panel and the first flap. The second flap is placed onto first flap. Still in another embodiment, either package has a third flap opposite the first flap. The third flap is folded onto the second flap and partially onto the first flap. In yet another embodiment, either package has a slit on the first flap to contain a portion of a distal edge of the third flap. In still another embodiment, either package has a folder. The folder contains a center panel; a recess in the folder panel to allow the needle or needles to be dispensed and with the double-armed suture, to allow the suture loop to be visible and to be severed; and at least a first flap adjacent the folder panel. The folder panel is placed onto the package first flap and the folder flap is placed onto the package panel. Alternatively, the folder panel is placed onto the package second flap and partially onto the first flap, or onto the third flap.

In yet another embodiment, the folder has a second flap opposite the folder first flap. The folder second flap is placed onto the folder first flap. In still yet another embodiment, the folder has a slit on the first flap and a coordinating tab on the folder second flap. The folder second flap is locked onto the folder first flap by the slit and coordinating tab.

An improved direct dispensing surgical suture package, particularly for microsurgical use, has also been invented. The package comprises a center panel; a foam receptacle affixed to said panel; a single-armed surgical suture with the needle engaged in the receptacle; and at least a first flap adjacent and placed onto said panel allowing said receptacle to be accessible. The improvement comprises a grid on the exterior package surface, whereby said suture can be dispensed from said package by disengaging and then pulling said needle, and said suture can be oriented and measured by placing it on said grid.

Other embodiments of the improved suture package have a second flap adjacent said panel and said first flap, said second flap placed onto said first flap; a third flap opposite said first flap, said third flap folded onto said second flap and partially onto said first flap; a slit on said first flap to contain a portion of a distal edge of said third flap; and a marking on said grid to show the distance between two parallel lines.

Another embodiment of the improved suture package is a folder containing a center panel and a plurality of flaps, at least two adjacent flaps of said folder being folded to form a pocket, a suture package described above placed into said pocket and the remaining flaps of said folder being folded. The package can be dispensed from said folder, said suture can be dispensed from said package by disengaging and then pulling said needle, and said suture can be oriented and measured by placing it on said grid.

Description of the Drawings

Figures 1 and 4 are perspective views and Figure 9 is a front view of alternative suture packages of this invention;

Figures 2, 5 and 10 are front views of the Figures 1, 4 and 9, respectively;

Figures 3 and 6 are perspective views and Figure 12 is a front view showing the folding sequence of the suture packages of Figures 2, 5 and 10, respectively;

Figure 7 is a perspective view, and Figures 13 to 15 are front views showing the folding sequence of folders useful with the suture packages of Figures 4 and 9, respectively;

Figure 8 is a perspective view showing the suture package of Figure 4 being loaded into the folder of Figure 7; and

Figure 11 is a perspective view showing the suture package of Figure 9 being removed from the folder of Figures 13 to 15.

The essence of this invention, described in conjunction with the attached drawings, comprises a folded paper holder for a double or single-armed ophthalmic suture. The holders for absorbable and nonabsorbable sutures are slightly different and are described separately below.

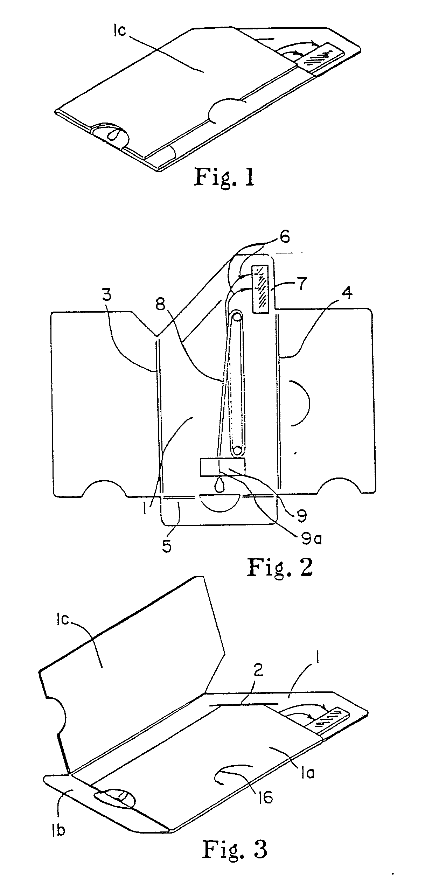

Figure 1 describes the preferred nonabsorbable suture package. The package can be contained in an exterior sealed envelope, for example as described in US 4,089,410 Figure 1 which is incorporated by reference. Preferably, the exterior envelope is composed of a gas pervious, bacteria-impervious spun bonded backing and transparent facing. Within this invelope is a suture holder, preferably composed of foldable 90 pound sterile offset paper. A label showing product information such as the type, size, and/or length of the suture and/or of the needles, and the manufacturer can be affixed to an exterior suture holder flap, for example flap 1c.

Figure 2 describes the suture holder after it has been removed from the envelope and opened along score lines 3, 4 and 5 to show construction and suture configuration. Although double score lines are described, it is to be understood that single score lines are also within the scope of this invention. The needles 6 are held in place by insertion into a first foam receptacle 7. Preferably, the first receptacle 7 is manufactured from a commercially available polyolefin foam, such as polyethylene or polypropylene. A double backed adhesive can be used to affix the foam receptacle to the suture holder.

The suture 8 is coiled on the center panel 1 of the suture holder. Preferably, the coil is in a helical or figure eight configuration. Generally, either coil configurations can be used. Also, other coil configurations, e.g. sinusoidal, may be used provided they allow dispensing of the suture or one-half of the suture from the holder without tangling. After coiling, the loop is formed in about the midpoint of the suture. The loop is held in place by insertion into a second foam receptacle 9. The second receptacle 9 contains a slit 9a to contain the loop. Optionally, the suture loop may be directed back to foam receptacle 7 and held in place by insertion into a slit within receptacle 7.

The suture holder of this invention is direct dispensing. That is, the suture holder does not have to be unfolded during use. After removing the suture holder from the exterior envelope, the user dispenses the suture by disengaging the needles from the first receptacle and then pulling them until the suture is removed from the holder. If only one half of the suture is required, the loop is severed prior to dispensing. A term such as "CUT" and/or a symbol can be printed on the suture holder or label in a visible location proximal to the suture loop. The user dispenses about one half of the suture by disengaging one of the needles from the first receptacle and then pulling on it until about one half of the suture is removed from the holder.

Figure 3 describes the folding sequence of the suture holder of Figure 2. Flap 1a is placed onto center panel 1. Slit 2 on center panel 1 is used to contain a distal corner of flap 1a. Flap 1b is then placed onto flap 1a. Flap 1b aids in containing the suture in the suture holder during loading and/or transporting. Flap 1b contains an opening to allow the loop to be visible and to be severed without unfolding the flap. Flap 1c is then placed onto flap 1b and partially onto flap 1a. Slit 16 of flap 1a is used to contain a distal edge of flap 1c.

Referring to Figure 3, when a distal edge of flaps 1a and 1c are adjacent to the score line(s) 5, a recess in the edge of both flaps is necessary to allow the suture loop to be acessible and to be severed without unfolding the flaps. Although a recess in the edge of both flaps is described, it is to be understood that alternative means of allowing the suture loop to be accessible and to be severed without unfolding the flap is within the scope of this invention. For example, a distal edge of flaps 1a and 1c can be adjacent to the second foam receptacle 9.

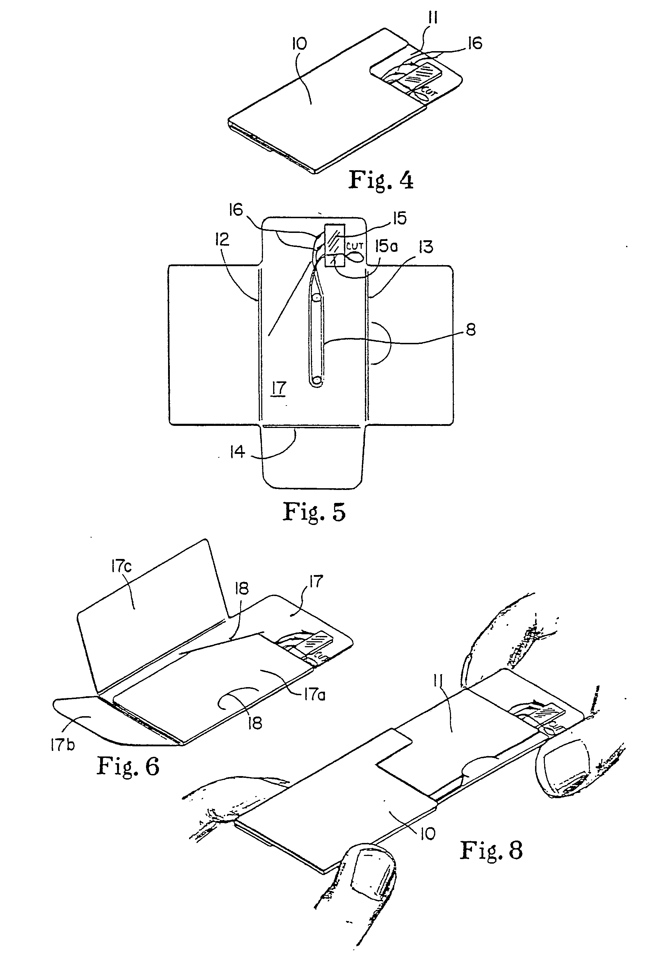

Figure 4 describes the preferred absorbable suture package. The package can be contained in an interior and then in an exterior sealed envelope. An adequate interior and exterior envelope useful with the package of this invention is described in the prior art.

Referred to Figure 4, the absorbable suture package contains a folder 10 and a suture holder 11. The folder 10 wraps around the suture holder to provide tear resistance to the suture holder. The folder 10 contains a recess to allow the needles 16 to be dispensed and to allow the suture loop to be accessible and to be severed.

Figure 5 describes the suture holder after it has been removed from the exterior and interior envelopes, and from the folder 10, and opened along score lines 12, 13 and 14 to show construction and suture configuration. Although double score lines are described, it is to be understood that single score lines are also within the scope of this insertion. The needles 16 are held in place by insertion into the receptacle 15. Preferably, the receptacle is manufactured from a commercially available polypropylene foam. A double backed adhesive can be used to affix the receptacle to the suture holder.

The suture 8 is coiled on the center panel 17 of the suture holder. Preferably, the coil is in a helical or figure eight configuration. Generally, either coil configuration can be used. Also, other coil configurations, e.g. sinusoidal, may be used provided they allow dispensing of the suture or one-half of the suture from the holder without tangling. After coiling, a loop is formed in about the midpoint of the receptacle 15. The receptacle contains a slit 15a to contain the loop.

The suture holder of this invention is direct dispensing. That is, the suture holder does not have to be unfolded nor does it have to be removed from the interior envelope and the folder during use. The absorbable suture holder is used by the user pulling open the exterior envelope. The interior envelope is then projected onto a sterile field. The interior envelope is then opened by a diagonal tear, exposing the receptacle 15. The user dispenses the suture by disengaging the needles from the receptacle and then pulling them until the suture is removed from the holder. If only one half of the suture is required, the loop is severed prior to dispensing. A term such as "CUT" and/or a symbol can be printed on the suture holder in a visible location proximal to the suture loop. The user dispenses one half of the suture by disengaging one of the needles from the receptacle and then pulling on it until about one half of the suture is removed from the holder.

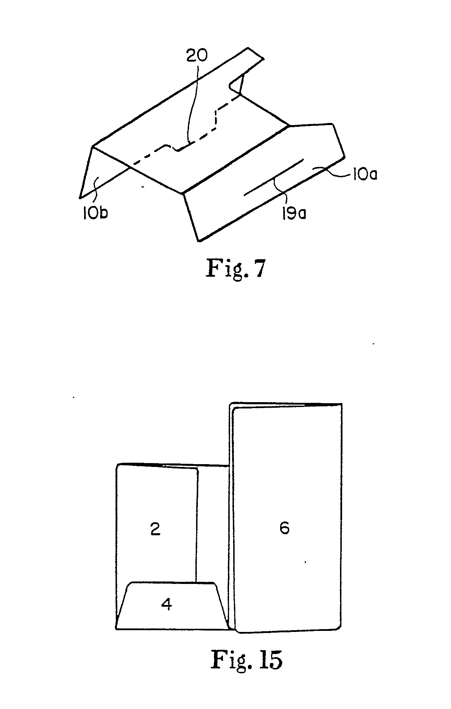

Figures 6 and 7 describe the folding sequence of the suture holder of Figure 5 and of the folder, respectively. Referring to Figure 6, flap 17a is placed onto center panel 17. Slit 18 on center panel 17 is used to contain a distal corner of flap 17a. Flap 17b aids in containing the suture in the suture holder during loading and/or transporting. Flap 17c is then placed onto flap 17b and partially onto flap 17a. Slit 18 on flap 17a is used to contain a distal edge of flap 17c.

Referring to Figure 7, flap 10a is folded such that it is contiguous with center panel 17. Flap 10b is then placed onto flap 10a. Slit 19a on flap 10a is used to contain tab 20 on flap 10b.

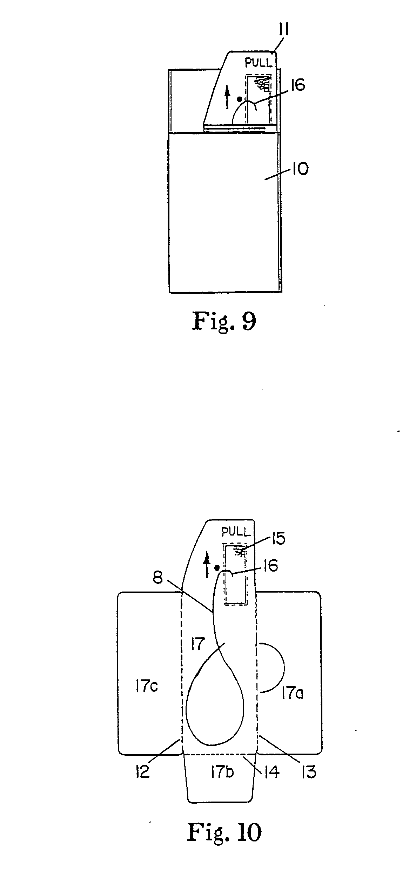

Figure 8 describes a method of loading the suture holder 11 into the folder 10. The folder is folded as described in conjunction with Figure 7 above. The suture holder is then slid into the folder such that the folder recess is adjacent to the needles and the loop. It is to be understood that alternative loading methods are within the scope of this invention. For example, the center panel of the holder 10 can be placed onto flap 17c. The holder 10 can then be folded as described in conjunction with Figure 7 above.

Figure 9 describes the preferred absorbable suture package for microsurgical use. The package can be contained in an interior and then in an exterior sealed envelope, which are known from the prior art.

Referring to Figure 9, the absorbable suture package contains a folder 10 and a suture holder 11. The folder 10 wraps around the suture holder to provide tear resistance to the suture holder. The height of the holder 11 is greater than the height of the front portion of the folder 10 to allow the needle 16 to be dispensed.

Referring to Figures 9 to 12, an optional embodiment is the word "PULL" and/or an arrow affixed to the upper portion of the holder 11 to indicate the direction for dispensing it from the folder 10. Another optional embodiment is a dot adjacent the butt end of the needle 16 to assist the user in locating the needle in the receptacle 15. Finally, the preferred receptacle 15 is shown in partial view. The needle 16 is therefore shown as being visible. It is to be understood however that the needle 16 is placed into and not on the receptacle 15.

Figure 10 describes the suture holder after it has been removed from the exterior and interior envelopes, the folder 10, and then opened along score lines 12, 13 and 14 to show construction and suture configuration. It is to be understood that single or double score lines separating the respective holder panels are within the scope of this invention. The needle 16 is held in place by insertion into the receptacle 15. Preferably, the receptacle is manufactured from a commercially available polypropylene foam. A double backed adhesive can be used to affix the receptacle 15 to the suture holder 11.

The suture 8 is loaded onto the center panel 17.

Any loading configuration may be used provided the dispensing of the suture from the holder is without tangling.

The suture package of this invention can be direct dispensing. That is, the suture holder 11 does not have to be unfolded or removed from the interior envelope or from the folder 10 during use. Preferably, however, the suture holder 11 is removed from the folder 10.

The absorbable suture holder 11 can be used by pulling open the exterior envelope. The interior envelope is then projected onto a sterile field. The interior envelope is then opened by a diagonal tear, exposing the needle 16 in the receptacle 15. The user can then dispense the suture 8 by disengaging the needle 16 from the receptacle 15 and then pulling it until the suture is removed from the package.

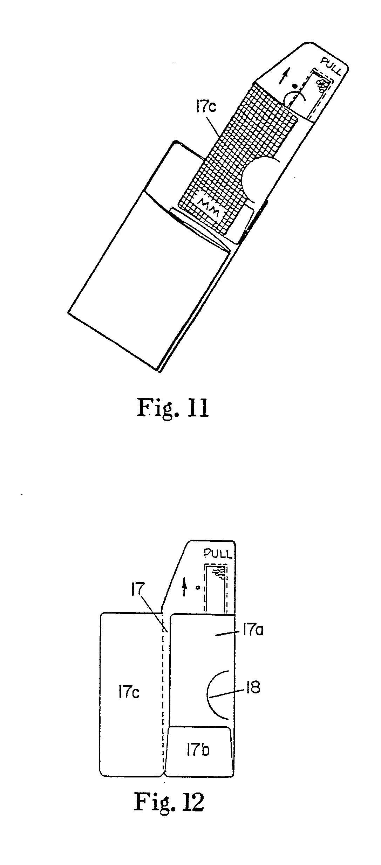

Alternatively, referring to Figure 11, the holder 11 can be removed from the folder 10. The user can then dispense the suture 8 from the holder 11 as described above. A grid on the exterior surface of the panel 17c can be used, for example, to orient and/or to measure the suture 8. A marking, for example "mm", can be used to show the actual distance between two parallel lines of said grid.

Figure 12 describes the folding sequence of the holder of Figure 10. Specifically, flap 17a is folded on score line 13 and placed onto center panel 17. Flap 17b is folded onto flap 17a. Flap 17b aids in containing the suture in the suture holder during loading and/or transporting. Flap 17c is then placed onto flap 17b and partially onto flap 17a. Slit 18a on flap 17a is used to contain an edge portion of flap 17c.

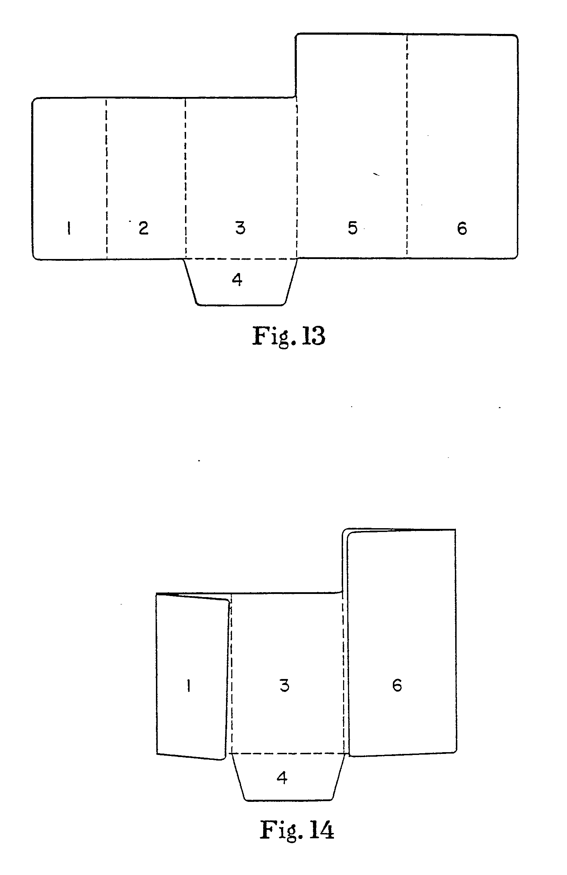

Figure 13 describes the folder after it has been removed from the exterior and interior envelopes and opened. It is to be understood that single or double score lines separating the respective folder panels can be within the scope of this invention. Figures 14 and 15 describe the folding sequence of the suture folder of Figure 13.

Referring to Figures 14 and 15, flap 1 is folded onto flap 2. Flap 1 is then folded onto the center panel 3. Flap 4 is folded onto flap 2. Flap 6 is then folded onto flap 5. Flap 6 is then folded onto flap 4 and partially onto flap 3.

Figure 11 describes a method of loading the suture holder 11 onto the folder 10. As described above, Figure 11 also describes a method of removing the folder 10 from the holder 11.

An alternative method of loading the holder 11 into the folder 10 as described in conjunction with Figures 14 and 15. The suture holder 11 can be placed in the pocket of the partially formed folder 10 between flaps 1 and 2. In this alternative method of loading, center panel 17 can be placed onto flap I of the folder 10. The folder 10 can then be completely assembled as described in Figures 14 and 15.

## Claims

1. A surgical suture package comprising a folded packet with a center panel (1, 17) and at least one flap (1a, 17a) adjacent to and placed onto said panel, and with at least one foam receptacle (7, 15) as a needle mounting means and optionally a suture loop retaining means, and further comprising a single-armed suture (8) with one needle (6) or a double-armed suture with two needles, said foam receptacle receiving the needle(s) and optionally a suture loop, characterized by the foam receptacle (7, 9; 15) fixed adjacent to the perimeter of at least one quadrant of the center panel (1; 17) and at least the first flap (1a, 17a) having a smaller perimeter than the perimeter of said panel (1; 17) allowing said needle(s) (6; 16) engaged in said receptacle (7; 15) and optionally said loop contained by said receptacle (9; 15) to be accessible without moving said flap (1a; 17a) relative to said panel (1; 17) whereby said suture (8) is directly dispensed from said package by disengaging and pulling one of said needles, optionally after severing said loop.

2. A suture package of claim 1, having a second flap (1b; 17b) adjacent said panel (1; 17) and said first flap (1a; 17a), the perimeter of said second flap being less than the perimeter of said first flap and second flap is placed onto said first flap.

3. A suture package of claim 2, having a third flap (1c; 17c) opposite to said first flap (1a; 17a), the perimeter of said third flap being at most equal to the perimeter of said first flap and at least greater than the perimeter of said second flap (1b; 17b), and said third flap is folded onto said second flap and partially onto said first flap.

4. A direct dispensing surgical suture package according to claim 1, further comprising a folder (10) containing a second center panel (3) and a plurality of side flaps, at least two adjacent side flaps of said folder (10) being folded to form a pocket, said suture package placed into said pocket and the remaining side flaps of said folder (10) being folded whereby said package can be dispensed from said folder (10); and a grid on the exterior suture package surface, whereby said suture (8) can be dispensed from said package by disengaging and then pulling said needle, and said suture (8) can be oriented and measured by placing it on said grid.

5. A suture package of claim 4, having a marking on said grid to show the distance between two parallel lines.

### Patentansprüche

1. Verpackung für chirurgisches Nahtmaterial, umfassend ein gefaltetes Paket mit einer mittleren Platte (1, 17) und mindestens einer Lasche (1a, 17a) neben der Platte und auf diese plaziert und mit mindestens einer Nadelaufnahme aus Schaum (7, 15) als eine Nadelhalterungseinrichtung und gegebenen falls mit einer Einrichtung zur Rückhaltung einer Nahtmaterialschlinge, und ferner umfassend ein einarmiges Nahtmaterial (8) mit einer Nadel (6) oder ein doppelarmiges Nahtmaterial mit zwei Nadeln, wobei die Nadelaufnahme aus Schaum die Nadel(n) und gegebenenfalls eine Nahtmaterialschlinge aufnimmt, dadurch gekennzeichnet, daß die Nadelaufnahme aus Schaum (7, 9; 15) neben dem Perimeter mindestens eines Quadranten der mittleren Platte (1; 17) befestigt ist und daß mindestens die erste Lasche (1a, 17a) einen kleineren Perimeter aufweist als der Perimeter der Platte (1; 17), so daß die Nadel(n) (6; 16) die in der Nadelaufnahme (7; 15) stecken und gegebenenfalls die durch die Nadelaufnahme (9; 15) gehaltene Schlinge zugänglich sind, ohne die Lasche (1a; 17a) relativ zu der Platte (1; 17) zu bewegen, wodurch das Nahtmaterial (8) aus der Verpakkung direkt abgegeben wird, indem man eine der Nadeln entnimmt und herauszieht, gegebenenfalls nach Zerschneiden der Schlinge.

2. Nahtmaterialverpackung nach Anspruch 1, mit einer zweiten Lasche (1b; 17b) neben der Platte (1; 17) und der ersten Lasche (1a; 17a), wobei der Perimeter der zweiten Lasche kleiner ist als der Perimeter der ersten Lasche und die zweite Lasche auf die erste Lasche plaziert ist.

3. Nahtmaterialverpackung nach Anspruch 2, mit einer dritten Lasche (1c; 17c) gegenüberliegend der ersten Lasche (1a; 17a), wobei der Perimeter der dritten Lasche höchstens gleich dem Perimeter der ersten Lasche ist und mindestens größer als der Perimeter der zweiten Lasche (1b; 17b) und wobei die dritte Lasche auf die zweite Lasche und teilweise auf die erste Lasche gefaltet ist.

4. Verpackung für chirurgisches Nahtmaterial zur direkten Abgabe nach Anspruch 1, ferner umfassend eine Faltmappe (10) enthaltend eine zweite mittlere Platte (3) und eine Vielzahl von Seitenlaschen, wobei mindestens zwei nebeneinanderliegende Seitenlaschen der Faltmappe (10) unter Ausbildung einer Tasche gefaltet sind, wobei die Nahtmaterialverpackung in der Tasche angeordnet ist und die restlichen Seitenlaschen der Faltmappe (10) in der Weise gefaltet sind, daß die Verpackung aus der Faltmappe (10) entnommen werden kann, und mit einem Gittermuster auf der Außenseite der Nahtmaterialverpackung, wobei das Nahtmaterial (8) aus der Verpackung abgegeben werden kann, indem man die Nadel entnimmt und anschließend herauszieht und wobei das Nahtmaterial (8) orientiert und abgemessen werden kann, indem man es auf das Gittermuster plaziert.

5. Nahtmaterialverpackung nach Anspruch 4, mit einer Markierung auf dem Gittermuster zur Anzeige des Abstands zwischen zwei parallelen Linien.

**Revendications**

1. Un emballage pour fils de sutures chirurgicales comprenant une trousse pliée ayant un panneau central (1, 17) et au moins un rabat (1a, 17a) adjacent audit panneau et placé sur celui-ci, au moins un réceptacle (7, 15) en mousse comme dispositif de fixation d'aiguille et, au choix, un moyen de retenue d'une boucle de fil de suture, et comprenant en outre un fil de suture (8) de simple longueur avec une aiguille (6) ou un fil de suture de double longueur avec deux aiguilles, le réceptacle en mousse recevant la ou les aiguilles, et, si on le désire, une boucle de fil de suture, caractérisé par le réceptacle en mousse (7, 9, 15) fixé au voisinage du périmètre d'au moins un quadrant du panneau central (1; 17) et au moins le premier rabat (1a, 17a) ayant un périmètre plus petit que le périmètre dudit panneau (1, 17) permettant à la ou aux aiguilles (6, 1) engagées dans ledit réceptacle (7, 15) et, si on le désire, à ladite boucle retenue par ce réceptacle (9, 15) d'être accessible sans déplcement du rabat (1a, 17a) par rapport au panneau (1, 17), de sorte que le fil (8) de suture chirurgicale est distribué directement à partir de cet emballage en dégageant et en tirant l'une des aiguilles, éventuellement, après avoir coupé la boucle.

2. Un emballage pour fils de sutures chirurgicales suivant la revendication 1, comportant un second rabat (1b, 17b) adjacent au panneau (1, 17) et au premier rabat (1a, 17a), le périmètre de second rabat étant inférieur au périmètre du premier rabat et le second rabat étant placé sur le premier rabat.

3. Un emballage pour fils de sutures chirurgicales suivant la revendication 2, comportant un troisième rabat (1c, 17c) opposé au premier rabat (1a, 17a), le périmètre de ce troisième rabat étant au plus égal au périmètre du premier rabat et au moins aussi grand que le périmètre du second rabat (1b, 17b), et ce troisième rabat étant replié sur le second rabat et partiellement sur le premier rabat.

4. Un emballage pour fils de sutures chirurgicales à distribution directe suivant la revendication 1, comprenant en outre une pochette (10) comportant un second panneau central (3) et plusieurs rabats latéraux, au moins deux rabats latéraux ajacents de cette pochette (10) étant repliés pour former une poche, l'emballage de fils de sutures chirurgicales étant disposé dans cette poche et les autres rabats latéraux de la pochette (10) étant repliés, de telle sorte que l'emballage peut être extrait de la pochette (10); et une grille sur la surface extérieure de l'emballage de fils de suture, de sorte que le fil de suture chirurgicale (8) peut être extrait de l'emballage en dégageant puis en tirant l'aiguille, et que le fil de suture chirurgicale (8) peut être orienté et mesuré en le plaçant sur la grille.

5. Emballage pour fils de sutures chirurgicales suivant la revendication 4, comportant une marque sur la grille pour montrer la distance entre deux barres parallèles.

Fig. 1

Fig. 2

Fig. 3

Fig. 4

Fig. 5

Fig. 6

Fig. 8

2

20

10b

10a

19a

**Fig. 7**

2

6

4

**Fig. 15**

Fig. 9

Fig. 10

17c

PULL

MM

Fig. 11

PULL

17

17a

17c

18

17b

Fig. 12

5

| 1 | 2 | 3 | 5 | 6 |

4

**Fig. 13**

| 1 | 3 | 6 |

4

**Fig. 14**